# EUROPÄISCHE PATENTSCHRIFT

(11) **EP 1 784 170 B1**
(45) Veröffentlichungstag und Bekanntmachung des Hinweises auf die Patenterteilung: **09.01.2008**
(21) Anmeldenummer: 05782111.8
(22) Anmeldetag: 01.09.2005
(51) Int. Cl.: A61K 9/70

(54) **ATRIUM PATCH**
ATRIUM PATCH
PATCH A ATRIUM

(30) Priorität: 01.09.2004 DE 102004042616
(43) Veröffentlichungstag der Anmeldung: 16.05.2007
(73) Patentinhaber: LABTEC GESELLSCHAFT FÜR TECHNOLOGISCHE FORSCHUNG UND ENTWICKLUNG MBH, 40764 Langenfeld (DE)
(72) Erfinder: VOLLMER, Ulrike, 41542 Dormagen (DE)
(74) Vertreter: Hansmann, Dierk
(86) Internationale Anmeldenummer: PCT/DE2005/001528
(87) Internationale Veröffentlichungsnummer: WO 2006/024284

(56) Entgegenhaltungen:
- EP-A- 1 034 780
- DE-A1- 19 856 101
- US-A- 3 699 963

## Beschreibung

Die Erfindung betrifft ein Atrium Patch, auch Buccalpflaster oder Mucosa-Adhäsivpflaster oder Orasan genannt.

Die Mundhöhle mit einer inneren Oberfläche von ca. 100 cm² stellt eine besonders vielfältige Funktion für den menschlichen Organismus dar. Zwar dient sie nur der Nahrungsaufnahme, stellt damit aber ein Organ dar, dass sich einerseits gegen die äußere Umwelt abgrenzen muss, andererseits aber schon zum inneren Organ gehört. Dies spiegelt sich wider in den verschiedenen epidermalen Bereichen. Seitlich liegt die Wangenschleimhaut (bukkale Applikationsseite genannt), oben liegt der Gaumen (palatale Applikationsseite genannt) und unten der Raum unter der Zunge (sublinguale Applikationsseite genannt). Des weiteren befindet sich im Mundraum die Zunge (keine Applikationsseite bisher, wichtiger Teil der sprachlichen Artikulation). Daneben enthält der Mund die für die Verdauung wichtigen Kauwerkzeuge (Zähne), die im Zahnfleisch verankert sind (auch als gingivale Applikationsseite bezeichnet). Exogen zugeführte Nahrungsmittel enthalten einerseits hohe mikrobielle Lasten als auch ideale Nährbedigungen für diese Mikroorganismen hinsichtlich Nahrungsquellen, Feuchte und Temperatur. Stark mikrobiell belastete Mundhöhlen zeichnen sich durch schmerzhafte Entzündungen, fehlende oder faulende Zähne, schlechten Atemgeruch und beeinträchtigte Nahrungsaufnahme aus. Zur Behebung bietet die Industrie zahlreiche Pflegemittel an, die neben Instrumenten wie Zahnbürsten, Zahnseiden, Mundduschen etc. auch aus Hilfsmitteln besteht wie Zahnpasten, Mundspülungen, Kaugummis und systemischen (z.B. antibiotischen) Arzneimitteln. Alle Hilfsmittel können entweder durch den Patienten selber oder durch Fachpersonal wie Zahnärzte angewendet werden.

Die benannten Applikationsseiten im Mundraum zeichnen sich allesamt durch die Gegenwart von Wasser aus, also im Falle der Applikation eines Pflasters muss die Adhäsion im Zustand der Nässe/Feuchtigkeit zustande kommen. In diesem Falle spricht man auch von Mucoadhäsion, weil die Haftung des Pflasters mit der Schleimhaut erfolgt, die mit Mucus ausgekleidet ist. Dabei wird die Mucoadhäsion als besondere Art der Bioadhäsion erachtet, da jede Adhäsion mit mindestens einer belebten Phase als Bioadhäsion bezeichnet wird. Die Dicke der nicht keratinisierten bukkalen Mucosa beträgt ca. 500 bis 800 µm, während die Schleimhäute des keratinisierten, sogenannten harten Gaumens und des Zahnfleisches etwa 100 bis 200 µm dick sind. Die Permeabilität der Mundschleimhäute ist höher als die der Epidermis der Haut, aber niedriger als die des Gastrointestinaltraktes. Die Sonderform der sublingualen Applikation soll hier nicht weiter interessieren, da sie bereits sehr gut untersucht ist, aber nur für wenige Anwendungen geeignet ist. Die geringe Dicke der Schleimhaut und die gute Durchblutung ermöglichen eine schnelle Resorption und gute Bioverfügbarkeit. Anders sieht es bei gingivaler und bukkaler Applikation aus. Bei gingivaler Applikation gilt schlechte Permeabilität, mäßige Durchblutung, aber gute Verweildauer für ein Pflaster. Bei bukkaler Applikation besteht etwas höhere Permeabilität und gute Durchblutung bei guter Verweildauer. Beide Applikationsseiten eigen sich für die Anwendung von Pflastern, die eine lokale Wirkung in der Mundhöhle entfalten sollen. Dies ist z.B. erforderlich bei Erkrankungen in der Mundhöhle. Dazu zählen die Applikation von Desinfektiva, Antibiotika Antimykotika, Virustatika, Corticoiden etc., teils auch in Form von Pflanzenextrakten, die bei Infektionen durch Bakterien, Pilzen und Viren auftreten wie Mundsoor, Herpes, Aphthen oder nach Zahnextraktionen. Des weiteren besteht die Notwendigkeit der Vorbeugung von Infektionen bei Läsionen, Ulzera, die z.B. gehäuft bei immun-suppressierten Personen auftreten. Ein weiterer Bedarf sind Lokalanästhetika für schmerzhafte Entzündungsprozesse oder Prothesendruckstellen. Es können aber auch Aromen, Naturstoffe oder Salze abgegeben werden, die Mundgeruch verbessern oder die Bildung von Plaque als Vorläufer von Gingivitis und Parodontitis mindern.

Es gibt noch weitere Körperstellen mit ganz ähnlichen Anforderungen wie die des Mundraums, die Rede ist von einer Körperöffnung (Orificium, Atrium) wie der Nase, der Scheide und des After als auch die umliegende Haut wie Lippen, Genitalien etc.. An allen diesen Pforten besteht eine verbindung von Innen und Außen der Organismus mit der Umwelt in Form eines Stoffaustausches, folglich haben diese Körperstellen eine mucosale Auskleidung der Epidermis und den Bedarf zur organgemäßen Therapie mit mucoadhäsiven Systemen.

Allen mucoadhäsiven Systemen wie dem hier zitierten Atrium Patch ist gemeinsam, dass sie an der Seite, die auf dem Mucus befestigt wird, Hilfsstoffe enthalten, die in diesem Milieu eine Adhäsion aufbauen können. Materialien dieser Art enthält u.a. die Publikation J. D. Smart et al., J. Pharm. Pharmacol. 1984, 36: 295-299. Es handelt sich durchweg um viskositätserhöhende Polymere, die sowohl natürlichen Ursprungs sein können wie Gelatine, Pektin, Tragant, Gummi arabicum, Chitosan, Hyaluronsäure, Alginate, Agar etc., also auch halbsynthetisch wie Celluslosederivate, v.a. Cellusloseether sowie vollsynthetische Polymere wie Polyacrylsäurederivate in verschiedenen Vernetzungsgraden oder Polyvinylalkohole, -pyrrolidone. Die Auswahl aus diesen Stoffen muss unter verschiedenen Gesichtspunkten gewählt werden. Zum einen müssen die Stoffe für die Herstellung eines Pflasters in der Lage sein, einen Film zu bilden. Weiter muss die mikrobiologische Reinheit und die mucosale Verträglichkeit gewährleistet sein sowie Verträglichkeit mit den eingesetzten Wirk- und Hilfsstoffen.

Bei der Verabreichung eines mucoadhesiven Systems muss die andere Seite durch eine nichtmucoadhesive Schicht abgedeckt werden, um ein Zusammenkleben (Inflation) der Körperöffnung zu vermeiden. Damit ergeben sich mehrer Möglichkeiten, das Atrium Patch zu konzipieren: ein lokales System gibt den Wirkstoff aus der mucoadhäsiven Schicht direkt an die überklebte Schleimhaut ab oder ein lokales System gibt den Wirkstoff aus der nichtmucoadhäsiven Schicht direkt an die Mundhöhle ab. Ein systemisches System hingegen gibt den Wirkstoff aus der mucoadhäsiven Schicht direkt an die überklebte Schleimhaut ab, um dort in den Blutkreislauf zu gelangen und dort eine zentrale Wirkung zu entfalten. Im Nachfolgenden wird im Schwerpunkt, aber nicht beschränkt, mehr auf die lokalen Systeme abgehoben.

Solche zweischichtigen Systeme sind bereits in der Schrift DE 198 56 1001 A1 hinreichend beschrieben.

So werden für die Adhäsivschicht synthetische Polyacrylsäurederivate mit der INCI-Bezeichnung Carbomer eingesetzt, wobei die verschieden stark quervernetzen Homopolymere Carbomer 971 und 974 sowie Carbomer 1342 (alle von BF Goodrich), keinen großen Unterschied hinsichtlich Quellung und Haftung gezeigt haben und auch Polycarbophil (BF Goodrich) gleichwertig eingesetzt werden kann. Die Rückschicht wurde aus Cellulosederivaten wie Hydroxypropylcellulose (HPMC), auch unter dem Handelsnamen Pharmacoat (Fa. Synthapharm) bekannt, gebildet, obgleich HPMC allein kaum Mucoadhäsivität besitzt und die Verblendung mit Cellulosederivaten (z.B. EP 275550 B1) üblicherweise die Haftungsfähigkeit von Carbomer verschlechtert.

Es wurde nun überraschend gefunden, dass die Verbindung beider Schichten erheblich verbessert werden kann durch den Zusatz an Gummi arabicum, allerdings muss dieser Polymer der Rückschicht, nicht der Adhesivschicht zugeführt werden, weil ansonsten das System des Atrium Patches nicht mehr gegeben ist.

Als wasserunlöslicher nichtionischer Celluloseether wird Ethylcellulose (Fa. Hercules, 48-49,5% Ethoxylgehalt, Substitutionsgrad 2,41 -2,51, niedrige viskosität) verwendet und als wasserlöslicher nichtionischer Celluloseether wird Hydroxypropylcellulose (Fa. Synthapharm, Pharmacoat 606, MG 11000), also ein vergleichbares Derivat wie in der Adhäsivschicht, eingesetzt.

Als wasserunlösliches anionisches Kolloid wird Carbopol 971 eingesetzt (Fa. Goodrich; Carbomer 971 CP), als wasserlösliches anionisches Kolloid wird das Salz der Arabinsäure (Gummi arabicum, Fa. Caelo) eingesetzt. Überraschenderweise wurde herausgefunden, dass die beste Verbindung beider Schichten erfolgt, indem die Ahesivschicht für die Mucosa das wasserunlösliche anionische Kolloid mit dem wasserlöslichen nichtionischen Celluloseether und die Rückschicht das wasserlösliche anionische Kolloid mit dem wasserunlöslichen Celluloseether erhält. Es ist dem Fachmann ersichtlich, dass man anionische und nichtionische Gummiarten mischen kann, nicht jedoch anionische und kationische. Nicht ersichtlich für den Fachmann war hingegen, dass gerade die Verhältnisse anionischer und nichtionischer Hydrokolloide in bestimmten indirekt proportionalen Verhältnissen in beiden Schichten enthalten sein müssen, um optimale Haftungen an mucosalen epidermalen Schichten von Körperöffnungen zu erreichen.

Als Wirkstoffe können verschiedene Mengen, entweder gelöst oder als Suspension eingesetzt werden, z.B. aber nicht ausschließlich, Antiseptika wie Chlorhexidin, Hexetidin, Cetylpyridinium etc. oder Antimykotika wie Miconazol, Clotrimazol, Econazol, Nystatin, Tolnaftat, Sulbentin, Itraconazol etc., oder Viustatika wie Amantadin, Aciclovir, Zidovudin etc., oder Antibiotika wie Tetracyclin, Metronidazol, Clindamycin etc., oder Lokalanästhetika wie Tetracain, Benzocain, Lidocain etc., oder Corticoide wie Triamcinolon, Hydrocortison etc., oder Salze des Fluorids, Kaliums, Zinks etc. oder Vitamine wie Ascorbinsäure, Tocopherol, Vitamin K, β-Carotin, Coenzym Q10 etc.

Es können auch wässrige oder alkoholische Extrakte von Pflanzen wie Echinacea, Sambucus, Matricaria, Thymus, Camiphora, Salix, Camelia, Salvia, Melissa, Myrtillum, Allium und weitere eingesetzt werden.

Die Herstellung muss so erfolgen, dass eine untrennbare Verbindung zwischen Adhäsiv- und Wirkschicht erfolgt. Da in den beiden Schichten keinerlei Bindemittel enthalten sind und die Laminierungstechnologie herkömmlicher Matrixpflaster angewendet werden soll und damit kein Kompressionsdruck zur Verfügung steht, konnte diese Verbindung überraschenderweise nur mit der Beschichtung der Lagen übereinander durch Überschichtung erzielt werden. Durch die besonderen Verhältnisse der eingesetzten und ausgewählten Hydrokolloide ist der Einsatz gleicher Phasen (beide wässrig oder beide organisch) nicht mehr notwendig. Trotzdem vermischen sich beide Schichten nicht, verbinden sich dennoch und sind im getrockneten Zustand nicht adhäsiv, so daß keine silikonisierten Schutzfolien während der Herstellung noch in der Verpackung erforderlich sind wie bei sonst üblichen Matrixpflastern.

Eine ethanolische Adhäsivschicht wird zuerst beschichtet, wobei dies auf Polyesterfolien, Glas oder anderen glatten Oberflächen erfolgen kann. Nach dem Trocknen wird die Wirkstoffschicht in einer ethanolischen Suspension überschichtet. Nach der endgültigen Trocknung kann das System ohne Probleme von der Trägergrundlage (Polyester, Glas etc.) abgezogen werden. Durch Stanzen erfolgt die Vereinzelung in Einzeldosen von 0,5 bis 2,5 cm², vorzugsweise in 0,8- 1,25 cm² sowie die Verpackung in luft- und lichtundurchlässige, dem Fachmann geläufige Peelpackungen.

Das Besondere dieser Erfindung besteht darin, dass die Verbindung der beiden Schichten immer dann besonders gut ist, wenn das Verhältnis der eingesetzten Hydrokolloide so erfolgt, dass nichtionische Polymere wie die Hydroxypropylmethyl- und Ethylcelluloseether und anionische Polymere wie Carbomer (Polyacrylsäure als Polyanion) und Gummi arabicum (Salz der Arabinsäure) im Verhältnis 1:1 bis 1:2, vorzugsweise 1:1,2 sind. Auf der anderen Seite ist es wichtig, dass das Verhältnis nichtionisches Hydrokolloid zu polyanionischem Hydrokolloid in der Adhesivschicht 1:2 bis 1:5, vorzugsweise 1:3 beträgt, in der Rückschicht aber umgekehrt, nämlich 2:1 bis 5:1, vorzugsweise aber 3:1.

Die genannten Hydrokolloide sind lediglich Ausführungsbeispiele. Der Fachmann kennt natürlich auch andere nichtionische Polymere als die hier genannten wie Celluloseether, z.B. Guar-Gummi, Johannisbrotkernmehl, Polyvinylpyrrolidon, Stärke etc. oder auch andere anionische Polymere als die hier genannten Carbomere und Gummi arabicum wie z.B. Agar-Agar, Alginsäure, Carboymethylcellulose, Carrageen, Gelatine Typ B, Karaya-Gummi, Pektin, Tragant, Polymethylvinylethermaleinanhydrid (Gantrez) etc.

### Beispiele:

### Erfindungsgemäßes Beispiel 1:

Um 3500 cm² eines erfindungsgemäßen Pflasterlaminats im Labormaßstab herzustellen, werden für die Adhäsivschicht 4,2 g Pharmacoat 606 und 2,8 g Glycerin anhydricum in 280 g Ethanol 96% gelöst, anschließend 0,42 g Titandioxid darin homogen suspendiert. Auf einem Rührer mit der Einstellung 300 rpm, 150°C werden 14 g Carbomer 971 auf die ethanolische Lösung aufgestreut, das System verschlossen und anschließend bei Raumtemperatur für 60 min im hochtourigen Mixer homogenisiert. Anschließendes langsames Rühren entfernt die eingearbeitete Luft. Es werden 1,4 g Erdbeeraroma (z.B. Dragoco) und 238,185 g eines wässrigen Pflanzenextraktes aus Trockenextrakt von Sambuccus nigra, Centella asiatica, Echinacea purpurea (9:0,8:0,2 wt, Gehalt Trockenrückstand 5,3%) zugegeben, gerührt und erhält ein homogenes, blasen- und klumpenfreies braunes Gel, welches auf Polyester (Fa. Hoechst, Hostaphan RN 100) mit einer Naßschichtdicke von 2000 µm ausgestrichen wird. Die Trocknung erfolgt bei ansteigender Temperatur von 40°C auf 60°C über 30 min. Für die Rückschicht werden 18 g Ethylcellulose auf 153 g Ethanol 96% aufgestreut und gerührt bei 700 rpm, bis alles gelöst ist. 0,36g Farbstoff E172 rot werden in 2ml Wasser gelöst. 0,45 g Titandioxid werden in 9 g Rizinusöl angerieben und 0,0,45 g unter Rühren zugegeben. Zum Schluss werden 6,75 g Gummi arabicum zugefügt und alles gerührt bei 300 rpm, bis eine homogene, hellrote, trübe, dünnflüssige Suspension entsteht. Diese wird auf die Adhäsivschicht mit einer Naßschichtdicke von 1000 µm beschichtet und ebenfalls bei ansteigender Temperatur von 40°C auf 60°C über 30 min getrocknet. Durch Stanzen resultieren mucoadhesive Pflaster der Größe 1,25 cm² elliptisch, die 4,5 mg Trockenextrakt enthalten und der Therapie von Gingivitis und Periodontitis dienen.

Das Verhältnis der nichtionogenen zu den anionischen Polymere beträgt im zitierten Falle 1: 1,2 ; das Verhältnis innerhalb der Adhäsivschicht ist 1: 3,3 ; dass der Rückschicht beträgt 2,7: 1.

### Erfindungsgemäßes Beispiel 2:

Um 2000 cm² eines erfindungsgemäßen Pflasterlaminats im Labormaßstab herzustellen, werden für die Adhäsivschicht 3,1 g Durotak 387-2353 (37,3% Feststoffeghalt) vorgelegt sowie 3,85 g Gantrez AN-139 sowie 3,5 g Aciclovir in 17,7 g Ethanol 96% unter Rühren gelöst. 0,12 g Titandioxid werden in 2 ml Ethanol 96% und ,8 g Glycerin anhydricum angerieben, 0,39 g Erdebeeraroma zugefügt und homogenisiert. Langsames Rühren entfernt die eingearbeitete Luft und ergibt eine homogenes, blasen- und klumpenfreies weißes Gel, welches auf Polyester (Fa. Hoechst, Hostaphan RN 100) mit einer Naßschichtdicke von 2000 µm ausgestrichen wird. Die Trocknung erfolgt bei ansteigend bei 40°C - 60°C über 30 min.

Für die Rückschicht werden 6,154 g Ethylcellulose in 52,3 g Ethanol 96 gelöst, 0,154 g Titandioxid werden in 3 g Rizinusöl angerieben. Letzteres und 2,3 g Gummi arabicum werden zur ethanolischen Ethylcellulose gegeben und durch rühren homogenisiert. Gegebenfalls wird Farbstoff und/oder Aroma zugegeben. Diese Lösung wird auf die Adhesivschicht mit einer Naßschichtdicke von 600 µm beschichtet und ebenfalls für 20 min bei 50°C getrocknet. Durch Stanzen resultieren mucoadhesive Pflaster der Größe 1,25 cm² elliptisch, die 5 mg Aciclovir enthalten und der Therapie von Herpes labialis und Herpes genitalis dienen.

### Erfindungsgemäßes Beispiel 3:

Das gemäß Beispiel 2 hergestellte, aber wirkstofffreie, Pflaster wurde in einem firmeninternen Tragetest auf die Eigenschaften der Applikation, der Haftungsdauer und der allgemeinen Convenience hin untersucht. Alle Teilnehmer hatten sich freiwillig bereiterklärt. Zum Zeitpunkt der Testung hatte keiner der Teilnehmer beiden Geschlechts eine Herpes labialis- Läsion, nur ein Teilnehmer von acht ist Herpespatient. Die Auswertung erfolgte nach Ausgabe eines Testpflaster anhand eines Fragebogens der Art "multiple choice". Der Test erfolgte innerhalb einer Woche. 77% der Teilnehmer fanden die Anwendung einfach. Die Anwendung sieht vor, dass die fettfreien Lippen mit der Zunge angefeuchtet werden und das Pflaster mit der weißen Seite auf die feuchte Lippe für 30 Sekunden angedrückt wird, bis eine feste Anhaftung erfolgt ist. 77% der Teilnehmer empfanden ein Fremdkörpergefühl auf der Lippe. 62,5% der Teilnehmer beurteilten die Haftung des Pflasters als gut und empfanden das Pflaster geschmacklich neutral. Die Tragedauer der Pflaster betrug im Mittel 5,6 Stunden; die minimale zeit war 1,3 Stunden, die maximale Zeit 19 Stunden. In jedem Falle wurde das Pflaster aktiv entfernt durch den Probanden, niemals fiel es durch Zufall ab. 50% der Teilnehmer begrüßten es, wenn das Pflaster noch dünner und oder flexibler sowie farbneutraler wäre. Diese Forderungen sind durch eine dünnere Beschichtung der Rückschicht als auch durch Änderung der Farbstoffe leicht möglich.

### Erfindungsgemäßes Beispiel 4:

Um 3500 cm² eines erfindungsgemäßen Pflasterlaminats im Labormaßstab herzustellen, werden für die Adhäsivschicht 4,2 g Pharmacoat 606 und 2,8 g Glycerin anhydricum in 280 g Ethanol 96% gelöst, anschließend 0,42 g Titandioxid darin homogen suspendiert. Auf einem Rührer mit der Einstellung 300 rpm, 150°C werden 14 g Carbomer 971 auf die ethanolische Lösung aufgestreut, das System verschlossen und anschließend bei Raumtemperatur für 60 min im hochtourigen Mixer homogenisiert. Anschließendes langsames Rühren entfernt die eingearbeitete Luft. Es werden 1,4 g Erdbeeraroma (z.B. Dragoco) und 238,185 g eines wässrigen Pflanzenextraktes aus Trockenextrakt von Sambuccus nigra, Centella asiatica, Echinacea purpurea (9:0,8:0,2 wt, Gehalt Trockenrückstand 5,3%) zugegeben, gerührt und erhält ein homogenes, blasen- und klumpenfreies braunes Gel, welches auf Polyester (Fa. Hoechst, Hostaphan RN 100) mit einer Naßschichtdicke von 2000 µm ausgestrichen wird. Die Trocknung erfolgt bei ansteigender Temperatur von 40°C auf 60°C über 30 min. Für die Rückschicht werden 18 g Ethylcellulose auf 153 g Ethanol 96% aufgestreut und gerührt bei 700 rpm, bis alles gelöst ist. 0,36g Farbstoff E172 rot werden in 2ml Wasser gelöst. 0,45 g Titandioxid werden in 9 g Rizinusöl angerieben und 0,0,45 g unter Rühren zugegeben. Alles wird gerührt bei 300 rpm, bis eine homogene, hellrote, trübe, dünnflüssige Suspension entsteht. Diese wird auf die Adhäsivschicht mit einer Naßschichtdikke von 1000 µm beschichtet und ebenfalls bei ansteigender Temperatur von 40°C auf 60°C über 30 min getrocknet. Durch Stanzen resultieren mucoadhesive Pflaster der Größe 1,25 cm² elliptisch, die 4,5 mg Trockenextrakt enthalten und der Therapie von Gingivitis und Periodontitis dienen. Diese Pflaster wird nach Verpackung in einen Beutel aus Verbundpackstoff bei Entnahme aus dem Beutel oder beim Applizieren auf der Schleimhaut sich delaminieren zwischen der Adhäsiv- und der Rückschicht.

Zur Veranschaulichung der in-vitro-Freisetzung von Beispiel 1:
Um zu verdeutlichen, daß es durch das richtige Verhältnis von anionischen und kationischen Polymeren nicht zu einer Bindung in der Wirkstoffschicht kommt, wurde die Freisetzung in vitro verglichen. Die Freisetzung erfolgte in der Blattrührer-Apparatur nach EP in 200 ml Phosphatpuffer bei 50 rpm, 37°C, wobei die Pflaster mit der Rückschicht auf das Klebeband der Scheibe fixiert werden. Die Proben wurden analysiert per HPLC (Temp. 40°C, Säule Interchrom (Interchim) Uptisphere UP5TF$15QS, 150 mm * 3,0 mm, Eluent A: 6,8 g ± 0,05 g Kaliumdihydrogenphosphat werden in 1000 ml Wasser gelöst und der pH-Wert mit 5 M KOH auf 4,6 eingestellt. Eluent B: 100 ml Milli-Q Wasser + 400 ml Acetonitril + 500 ml Methanol, werden gut durchmischt und mindestens 10 min ultrabeschallt; Gradient: 25% Eluent B, linear auf 60% steigend Eluent B von 0 auf 15 min. steigend, ab 15 min wieder auf 25% Eluent B fallend; Detektion Isoquercetrin bei 254 nm, Flußrate 0,8 ml/min, Injektionsvolumen 20 µl, Stop bei 15 min).

Alle Angaben in mg/ 1,25 cm² ; Mittelwert von 6 Proben:

| zeit [h] | Beispiel 1 |
|---|---|
| 0 | 0 |
| 1 | 0,041 mg Isoquercitrin = 91% |

Wie aus der Tabelle ersichtlich, ist die Freisetzung in dem erfindungsgemäßen Beispiel nach 1 Stunde nahezu komplett. Auch trotz der geringen Mengen der quantifizierten Markersubstanz Isoquercetin (Isoquercitrin) lässt sich das analytisch gut charackterisieren.

Als qualitätsbestimmende Parameter wurden die folgenden untersucht:
Flächengewicht: aus 20 Pflastern per Wägung als Mittelwert, Angabe in mg/cm²
Trocknungsverlust: an 3 Pflastern gemäß Ph. Eur. 2.2.32, 24 h Excikator vakuumiert bei RT, Mittelwert %
Haftungskraft: in-vitro an 3 Pflastern, 2h bei 70% hydratisiert, Frank-Gerät, "Peel", Stahlplatte, in N
Freisetzung in-vitro: Ph. Eur. 2.9.4 wie oben beschrieben

Gehalt: n=10 für Gleichförmigkeit; Analyse erfolgt nach 4 h Extraktion auf dem Kreisschüttler bei RT in 20 ml Phosphatpuffer per HPC gegen externen Standrad wie bei der Freisetzung beschrieben. Es wird der Gehalt von 45 µg per Pflaster an der Markersubstanz Isoquercetin herangezogen.

## Patentansprüche

1. Pflasterzubereitung für die Administration von Wirkstoffen in und an Körperöffnungen wie z.B. die Mundhöhle, die Lippen, die Genitalien etc., bestehend aus einer Adhäsivschicht und einer Rückschicht, letztere enthält eine Matrix aus einem nichtionischen und einem anionischen Hydrokolloid im Verhältnis 2:1 bis 5:1, die Adhesivschicht hingegen ein nichtionisches und ein anionisches Hydrokolloid im umgekehrt proportionalen Verhältnis von 1:2 bis 1:5.

2. Pflasterzubereitung nach Anspruch 1, **dadurch gekennzeichnet, dass** es sich bei der Adhäsivschicht um eine Matrix aus einem nichtionischen und einem anionischen Hydrokolloid im Verhältnis 3:1 , bei der Adhesivschicht hingegen ein nichtionisches und ein anionisches Hydrokolloid im umgekehrt proportionalen Verhältnis von 3:1 handelt.

3. Pflasterzubereitung nach Anspruch 1 bis 2, **dadurch gekennzeichnet, dass** sich bei den nichtionischen Hydrokolloiden um einen Polymer wie Hydroxypropylcellulose und Ethylcellulose handelt.

4. Pflasterzubereitung nach Anspruch 1 bis 2, **dadurch gekennzeichnet, dass** sich bei den nichtionischen Hydrokolloiden um einen Polymer wie Guar-Gummi, Johannisbrotkernmehl, Polyvinylpyrrolidon, Stärke etc handelt.

5. Pflasterzubereitung nach Anspruch 1 bis 2, **dadurch gekennzeichnet, daß** sich bei den anionischen Hydrokolloiden um einen Polymer wie Carbomer (Polyacrylsäure) und Gummi arabicum (Salz der Arabinsäure) handelt.

6. Pflasterzubereitung nach Anspruch 1 bis 2, **dadurch gekennzeichnet, dass** sich bei den anionischen Hydrokolloiden um einen Polymer wie Agar-Agar, Alginsäure, Carboymethylcellulose, Carrageen, Gelatine Typ B, Karaya-Gummi, Pektin, Tragant, Polymethylvinylether-maleinanhydrid etc. handelt.

7. Pflasterzubereitung nach Anspruch 1 bis 2, **dadurch gekennzeichnet, dass** das Verhältnis der nichtionischen und anionischen Hydrokolloide im Gesamtpflaster 1:1 bis 1:5 ist.

8. Pflasterzubereitung nach Anspruch 1 bis 2, **dadurch gekennzeichnet, dass** das Verhältnis der nichtionischen und anionischen Hydrokolloide im Gesamtpflaster 1:1 bis 1:2 ist.

9. Pflasterzubereitung nach Anspruch 1 bis 2, **dadurch gekennzeichnet, dass** die Adhäsivschicht und die Rückschicht untrennbar miteinander verbunden sind, was durch die beschriebene Verhältnismäßigkeit der nichtionischen und anionischen Polymere erfolgt.

10. Pflasterzubereitung nach Anspruch 1 bis 2, **dadurch gekennzeichnet, dass** es sich bei den Wirkstoffen zum Beipspiel aber nicht ausschließlich um Antiseptika wie Chlorhexidin, Hexetidin, Cetylpyridinium etc. und/oder Antimykotika wie Miconazol, Clotrimazol, Econazol, Nystatin, Tolnaftat, Sulbentin, Itraconazol etc., und/oder Virustatika wie Amantadin, Aciclovir, Zidovudin, Imiquimod etc., und/oder Antibiotika wie Tetracyclin, Metronidazol, Clindamycin etc., und/oder Lokalanästhetika wie Tetracain, Benzocain, Lidocain etc., und/oder Corticoide wie Triamcinolon, Hydrocortison etc., oder Salze des Fluorids, Kaliums, Zinks etc. und/oder Vitamine wie Ascorbinsäure, Tocopherol, Vitamin K, β-Carotin, Coenzym Q10 handelt.

11. Pflasterzubereitung nach Anspruch 1 bis 2, **dadurch gekennzeichnet, dass** es sich bei den Wirkstoffen zum Beipspiel aber nicht ausschließlich um wässrige und/oder alkoholische Extrakte von Pflanzen wie Echinacea, Sambucus, Matricaria, Thymus, Camiphora, Salix, Camelia, Salvia, Melissa, Myrtillum, Allium etc. handelt.

12. Pflasterzubereitung nach Anspruch 1 bis 11, **dadurch gekennzeichnet, dass** sowohl in der Adhäsivschicht als auch in der Rückschicht übliche Hilfsstoffe wie Aromen, Süßstoffe, Farbstoffe, Stabilisatoren, Antioxidantien, Konservierungsmittel, oberflächenaktive Substanzen, Füllstoffe und andere Hilfsmittel zugesetzt werden können.

## Claims

1. A dressing preparation for administering active ingredients in and on body orifices such as for example the oral cavity, the lips, the genitals etc, consisting of an adhesive layer and a backing layer, the latter contains a matrix of a nonionic and an anionic hydrocolloid in a ratio of 2:1 to 5:1, while the adhesive layer on the other hand contains a nonionic and an anionic hydrocolloid in the inversely proportional ratio of 1:2 to 1:5.

2. A dressing preparation according to claim 1, **characterised in that** the adhesive layer comprises a matrix of a nonionic and an anionic hydrocolloid in the ratio 3:1, while the adhesive layer on the other hand contains a nonionic and an anionic hydrocolloid in the inversely proportional ratio of 3:1.

3. A dressing preparation according to claims 1 to 2, **characterised in that** the nonionic hydrocolloids are a polymer such as hydroxypropylcellulose and ethylcellulose.

4. A dressing preparation according to claims 1 to 2, **characterised in that** the nonionic hydrocolloids are a polymer such as guar gum, locust bean flour, polyvinylpyrrolidone, starch etc.

5. A dressing preparation according to claims 1 to 2, **characterised in that** the anionic hydrocolloids are a polymer such as carbomer (polyacrylic acid) and gum arabic (salt of arabic acid).

6. A dressing preparation according to claims 1 to 2, **characterised in that** the anionic hydrocolloids are a polymer such as agar-agar, alginic acid, carboxymethylcellulose, carrageenan, type B gelatine, karaya gum, pectin, tragacanth, polymethyl vinyl ether/maleic anhydride etc.

7. A dressing preparation according to claims 1 to 2, **characterised in that** the ratio of nonionic and anionic hydrocolloids in the complete dressing is 1:1 to 1:5.

8. A dressing preparation according to claims 1 to 2, **characterised in that** the ratio of nonionic and anionic hydrocolloids in the complete dressing is 1:1 to 1:2.

9. A dressing preparation according to claims 1 to 2, **characterised in that** the adhesive layer and the backing layer are inseparably joined together, this being achieved thanks to the described proportionality of the nonionic and anionic polymers.

10. A dressing preparation according to claims 1 to 2, **characterised in that** the active ingredients are for example but not exclusively antiseptics such as chlorhexidine, hexetidine, cetylpyridinium etc. and/or antimycotics such as miconazole, clotrimazole, econazole, nystatin, tolnaftate, sulbentin, itraconazole etc, and/or virostatic agents such as amantadine, aciclovir, zidovudine, imiquimod etc, and/or antibiotics such as tetracycline, metronidazole, clindamycin etc, and/or local anaesthetics such as tetracaine, benzocaine, lidocaine etc, and/or corticoids such as triamcinolone, hydrocortisone etc, or salts of fluoride, potassium, zinc etc. and/or vitamins such as ascorbic acid, tocopherol, vitamin K, β*-*carotene, coenzyme Q10.

11. A dressing preparation according to claims 1 to 2, **characterised in that** the active ingredients are for example but not exclusively aqueous and/or alcoholic extracts of plants such as Echinacea, Sambucus, Matricaria, Thymus, Camiphora, Salix, Camellia, Salvia, Melissa, Myrtillum, Allium etc.

12. A dressing preparation according to claims 1 to 11, **characterised in that** conventional auxiliaries such as aromas, sweeteners, dyes, stabilisers, antioxidants, preservatives, surface-active substances, fillers and other auxiliary substances may be added both to the adhesive layer and to the backing layer.

## Revendications

1. Préparation d'emplâtre pour l'administration de substances actives dans et sur les orifices du corps, comme, par exemple, la cavité buccale, les lèvres, les organes génitaux etc., qui est formée par une couche adhésive et par une couche arrière, cette dernière comprenant une matrice qui est formée par un hydro colloïde non ionique et par un hydrocolloïde anionique, dans un rapport proportionnel de 2 :1 à 5 :1, la couche adhésive comprenant, par contre, un hydrocolloïde non ionique et un hydrocolloïde anionique, dans un rapport proportionnel opposé de 1 : 2 à 1 : 5.

2. Préparation d'emplâtre selon la revendication 1, **caractérisée en ce que** la couche adhésive est une matrice qui comprend un hydrocolloïde non ionique et un hydrocolloïde anionique, dans un rapport proportionnel de 3 : 1, tandis que la couche de dos est composée d'un hydrocolloïde non ionique et d'un hydrocolloïde anionique, dans un rapport proportionnel opposé de 1 : 3.

3. Préparation d'emplâtre selon les revendications 1 à 2, **caractérisée en ce que** les hydrocolloïdes non ioniques sont des polymères, comme de la cellulose hydroxy-propylique et de la cellulose éthylique.

4. Préparation d'emplâtre selon les revendications 1 à 2, **caractérisée en ce que** les hydrocolloïdes non ioniques sont des polymères, comme de la gomme de guar, de la farine de caroube, du pyrrolidon de polyvinyle, de l'amidon etc.

5. Préparation d'emplâtre selon les revendications 1 à 2, **caractérisée en ce que** les hydrocolloïdes anioniques sont des polymères, comme carbomère (acide polyacrylique) et gomme arabique (sel de l'acide arabique).

6. Préparation pour emplâtre selon les revendications 1 à 2, **caractérisée en ce que** les hydrocolloïdes anioniques sont des polymères, comme l'agar-agar, l'acide alginique, la cellulose carboxyméthylique, du carragheen, de la gélatine de type B, de la gomme de sterculia, de la pectine, de la gomme adragante, de l'acide maléique de polyméthylvinyle etc.

7. Préparation pour emplâtre les revendications 1 à 2, **caractérisée en ce que** le rapport proportionnel des hydrocolloïdes non ioniques et anioniques dans l'emplâtre entier est de 1 : 1 à 1 : 5.

8. Préparation pour emplâtre selon les revendications 1 à 2, **caractérisée en ce que** le rapport proportionnel des hydrocolloïdes non ioniques et anioniques dans l'emplâtre entier est de 1 : 1 à 1 : 2.

9. Préparation pour emplâtre selon les revendications 1 à 2, **caractérisée en ce que** la couche adhésive et la couche de dos sont reliées ensemble de manière inséparable ce qui résulte des rapports proportionnels décrits des polymères non ioniques et anioniques.

10. Préparation d'emplâtre selon les revendications 1 à 2, **caractérisée en ce que** les substances actives sont, par exemple, mais pas exclusivement, des antiseptiques, comme chlorhéxidine, hexétidine, cétylpyridinium etc., et / ou des antimycotiques, comme miconazole, clotrinazole, éconazole, nystatine, tolnaftate, sulbentine, itraconazole etc. et / ou des virostatiques, comme amantadin, aciclovir, zidovudine, imiquimode etc., et / ou des antibiotiques, comme tétracycline, métronidazole, clindamycine etc., et /ou des anesthésiques locaux, comme tétracaïne, benzocaïne, lidocaïne etc., et / ou des corticoïdes, comme triamcinolone, hydrocortisone etc., ou des sels de fluorite, de potassium, de zinc etc., et / ou des vitamines, comme acide ascorbique, tocophérol, vitamine K, β-carotène, coenzyme Q10.

11. Préparation d'emplâtre selon l'une des revendications 1 à 2, **caractérisée en ce que** les substances actives sont, par exemple, mais pas exclusivement, des extraits aqueux, et / ou alcooliques de plantes, comme échinacea, sambucus, matricaria, thym, camiphora, salix, camélia, salvia, mélisse, myrtille, allium etc.

12. Préparation d'emplâtre selon l'une des revendications 1 à 11, **caractérisée en ce que** aussi bien dans la couche adhésive que dans la couche de dos, on peut ajouter des substances auxiliaires, courantes, comme des arômes, des édulcorants, des colorants, des stabilisateurs, des antioxydants, des conservateurs, des substances actives en surface, des charges et d'autres adjuvants.
